# EUROPEAN PATENT SPECIFICATION

(11) **EP 1 379 299 B1**
(45) Date of publication and mention of the grant of the patent: **11.06.2008**
(21) Application number: 01274126.0
(22) Date of filing: 13.04.2001
(51) Int. Cl.: A61M 5/32, A61M 5/46

(54) **PREFILLABLE INTRADERMAL INJECTOR**
VORGEFÜLLTER INTRADERMALER INJEKTOR
INJECTEUR INTRADERMIQUE A PREREMPLISSAGE

(43) Date of publication of application: 14.01.2004
(73) Proprietor: Becton Dickinson and Company, Franklin Lakes, New Jersey 07417-1880 (US)
(72) Inventor: ALCHAS, Paul, G., Wayne, NJ 07470 (US); GUILLERMO, Carlos, E., Clinton, CT 06413 (US); KORISCH, Marina, S., Wayne, NJ 07470 (US)
(74) Representative: Selting, Günther
(86) International application number: PCT/US2001/012247
(87) International publication number: WO 2002/083212

(56) References cited:
- WO-A-99/25402
- US-A- 3 890 971

## Description

### FIELD OF THE INVENTION

The present invention relates generally to a prefillable delivery device for delivering substances such as drugs, vaccines and the like used in the prevention, diagnosis, alleviation, treatment or cure of diseases, and more specifically relates to a drug delivery device having a needle cannula and a limiter for engaging the surface of the skin and limiting penetration of the tip of the needle cannula into the skin. More specifically, the present invention relates to a limiter capable of fixing the orientation of the needle cannula in a generally perpendicular plane to the skin engaging surface of the limiter and capable of concealing the needle cannula subsequent to administering the intradermal injection.

### BACKGROUND OF THE INVENTION

Intradermal injections are used for delivering a variety of substances. Many of these substances have proven to be more effectively absorbed into or react with the immune response system of the body when injected intradermally. Recently, clinical trials have shown that hepatitis B vaccines administered intradermally are more imunogenic if administered intramuscularly. In addition, substances have been injected intradermally for diagnostic testing, such as, for example using what is known in the art as the "Mantoux test" to determine the immunity status of the animal against tuberculosis and the immediate hypersensitivity status of Type I allergic diseases.

An intradermal injection is made by delivering the substance into the epidermis and upper layers of the dermis. Below the dermis layer is subcutaneous tissue (also sometimes referred to as the hypodermis layer) and muscle tissue, in that order. There is considerable variation in the skin thickness both between individuals and within the same individual at different sites of the body. Generally, the outer skin layer, epidermis, has a thickness between 50-200 microns, and the dermis, the inner and thicker layer of the skin, has a thickness between 1.5-3.5 mm. Therefore, a needle cannula that penetrates the skin deeper than about 3.0 mm has a potential of passing through the dermis layer of the skin and making the injection into the subcutaneous region, which may result in an insufficient immune response, especially where the substance to be delivered intradermally has not been indicated for subcutaneous injection. Also, the needle cannula may penetrate the skin at too shallow a depth to deliver the substance and result in what is commonly known in the art as a "wet injection" because of reflux of the substance from the injection site.

The standard procedure for making an intradermal injection is known to be difficult to perform, and therefore dependent upon experience and technique of the healthcare worker. This procedure is recommended to be performed by stretching the skin, orienting the bevel of a 26 Gauge short bevel needle cannula upwardly and inserting the needle cannula to deliver a volume of 0.5 ml or less of the substance into the skin of an animal with the needle cannula being inserted into the skin at an angle varying from around 10-15 degrees relative to the plane of the skin to form a blister or wheal in which the substance is deposited or otherwise contained. Accordingly, the technique utilized to perform the standard intradermal injection is difficult and requires the attention of a trained nurse or medical doctor. This procedure also makes it essentially impossible to self-administer an intradermal injection. Inserting the needle to a depth greater than about 3.0 mm typically results in a failed intradermal injection because the substance being expelled through the cannula will be injected into the subcutaneous tissue of the animal. Further, the present method is not suitable for self-administration of intradermal injections.

Further, with the advent of viral infections that are transferred through contact with bodily fluids, it is desirable to enclose a needle cannula subsequent to administering an injection. Preferably, a needle assembly should include a mechanism that is capable of enclosing a needle cannula immediately subsequent to administering the injection. If a needle is left uncovered for even a short period of time after administering an injection, such as, for example, while trying to reattach a needle cap, a biohazard exists. Copending United States Patent Application No. 09/417,671 discloses needle assemblies having a limiter to facilitate administering an intradermal injection, the disclosed assemblies are not capable of concealing or enclosing the needle cannula after administering the injection. Therefore, it is desirable to provide a needle assembly with an integral device that is simply designed, easy to use, and readily available immediately after administering an injection.

A prefillable intradermal injector corresponding to the first part of claim 1 is known from U.S. 3,890,971. This injector comprises a housing containing a reservoir and a plunger moveable relative to the housing for advancing a stopper that is moveable within the reservoir for injecting a liquid through a hollow needle. The hollow needle is covered by a cap. For injecting the liquid, the user has to press the housing forward so that the needle point extends beyond a front wall of the cap. Thereafter, the user has to push the plunger in forward direction to eject the liquid from the reservoir. Then, the user has to move the plunger backwards until the plunger reaches a locking position. Then, the user has to pull the cap out of the housing so that the cap reaches a locking position relative to the housing, wherein the cap covers the hollow needle.

WO 99/25402 discloses a needle arrangement for an injection device. The device comprises a cap which can slide on the cannula carrier. The cap comprises a though hole for the cannula and in its proximal end position, it covers the cannula. A pressure spring is provided between the cannula carrier and the cap for moving the cap into its proximal end position. The injection device needs an own plunger that has to be moved manually irrespective of the handling of needle arrangement.

Accordingly, there has been a need for a needle assembly of intradermal injection device providing the ability to perform an intradermal injection of substances which overcomes the problems and limitations associated with conventional needle assemblies. Further, there has been a need to provide the needle assembly with an enclosure device that is capable of enclosing a needle cannula immediately subsequent to administering the intradermal injection. The combination of these two features on the same needle assembly would provide the ability to both reduce the probability of error and pain caused from the intradermal injection and to enclose the needle cannula after the injection has been administered.

### SUMMARY OF THE INVENTION AND ADVANTAGES

It is an object of the invention to provide a prefillable intradermal injector that is simply designed and easy to use.

The intradermal injector of the present invention is defined by claim 1. Accordingly, it is characterized in that the sleeve surrounds and is slidable relative to the reservoir along the axis and the sleeve is fixed relative to the stopper whereby movement of the sleeve causes the stopper to move through the reservoir to expel the substance from the reservoir through the needle cannula.

In contrast to the needle assemblies discussed above, the present invention both enables the administration of an intradermal injection utilizing a simplified method that reduces the probability of error and also enables the user to conceal the needle immediately after administering the injection. Specifically, The prefillable intradermal injector of the present invention for use in intradermally injecting substances into the skin of an animal includes a prefillable reservoir adapted to store a substance and having an outlet port at a first end enabling the substance to be expelled from the reservoir and a stopper in a second open end, a needle cannula defining an axis and the needle cannula in fluid communication with the reservoir to receive the substance from the outlet port and having a forward tip adapted to penetrate the skin of an animal, a limiter surrounding the needle cannula, the limiter including a generally flat skin engaging surface extending in a plane generally perpendicular to an axis of the needle cannula, and a sleeve having a first end and a second end, the sleeve surrounding and slidable relative to the reservoir along the axis and the sleeve fixed relative to the stopper whereby movement of the sleeve causes the stopper to move through the reservoir to expel the substance from the reservoir through the needle cannula.

In the preferred embodiment of the prefillable intradermal injector, the limiter is moveable axially relative to the needle cannula from a retracted position wherein the forward tip of the needle cannula extends through the limiter to an extended position wherein the needle cannula forward tip is enclosed within the limiter. Also, the forward tip of the needle cannula extends through the limiter beyond the skin engaging surface no more than from about 0.5 mm to about 3.0 mm in the retracted position of the limiter.

In addition, in the preferred embodiment, the limiter is moveable axially relative to the reservoir from a first position wherein the forward tip of the needle is located within the limiter to a second position wherein the needle extends through the limiter beyond the skin engaging surface no more than from about 0.5 to 3.0 mm, with the limiter including an axial opening therethrough receiving the forward tip of the needle cannula and the opening includes pierceable insert sealing the axial opening, and the forward tip of the needle cannula preferably extending beyond the skin engaging surface no more than from about 1.0 to 2.0 mm, and more preferably extending beyond the skin engaging surface about 1.5 mm ± 0.2 to 0.3 mm. Also, the forward tip of the needle cannula pierces the insert when the limiter is located in the second position thereby exposing the forward tip enabling administration of the intradermal injection.

Also, in the preferred embodiment, the limiter includes a locking arm and the sleeve includes a catch engaging the locking arm when the limiter is located in the extended position enclosing the forward tip of the needle cannula, with the locking arm including a hook disposed upon a distal end and the catch includes a slot disposed in the sleeve and the hook engaging the slot when the limiter is located in the extended position. Further, the prefillable container includes a stop to terminate movement of the limiter at the second position when moving the limiter between the first position and the second position. Still further, the prefillable intradermal injector includes a spring biasing the limiter to move from the retracted position to the extended position, with the spring preferably coiled around the reservoir. The sleeve includes a stopper engaging portion, with stopper engaging portion including a plunger rod operably connected to the second end of the sleeve.

The limiter limits penetration of the forward tip of the needle cannula into the dermis layer of the skin during the step of moving the limiter in the first direction, with penetration of the forward tip of the needle cannula limited to about 0.5 to 3.0 mm, and preferably limited to about 1.0 to 2.0 mm or 1.5 mm ± 0.2 to 0.3 mm.

Alternatively, the prefillable intradermal injector of the present invention includes a reservoir prefillable with a substance having a generally closed proximal end and an open distal end, a needle cannula having a distal end in fluid communication with the substance in the reservoir extending through the proximal end and an exposed forward tip, a stopper slideably received in the open distal end of the reservoir moveable in the reservoir to expel the substance through the forward tip of the needle cannula, and a limiter having a distal end slideably supported around the reservoir and moveable relative to the reservoir and a generally closed proximal end including a flat skin engaging surface having an opening therethrough receiving the forward tip of the needle cannula therethrough as the limiter moves relative to the reservoir and the limiter limiting extension of the forward tip of the needle cannula beyond the skin engaging surface a distance of more than 3.0 mm.

In this embodiment of the prefillable intradermal injector, the proximal end of the reservoir engages a surface of the limiter as the limiter is moved toward the reservoir, thereby limiting movement of the limiter relative to the reservoir. In addition, the intradermal injector includes a sleeve surrounding the reservoir operably fixed to the stopper whereby movement of the sleeve relative to the reservoir moves the stopper through the reservoir. Also, the opening through the proximal end of the limiter includes a pierceable closure and the forward tip of the needle cannula pierces the closure as the limiter is moved toward the reservoir, with the forward tip of needle cannula extends through the limiter beyond the skin engaging surface no more than from about 1.0 to 2.0 mm, and preferably 1.5 ± 0.2 to 0.3 mm. Further, the intradermal injector includes a spring biased between the reservoir and the limiter moving the limiter to an extended position enclosing the forward tip of the needle cannula following injection.

The present invention provides the desirable features set forth above that are not presently included on the same needle assembly. The limiter facilitates making an intradermal injection at a generally perpendicular angle to the skin of the animal and then also conceal or enclose the needle subsequent to administering the injection.

### BRIEF DESCRIPTION OF THE DRAWINGS

Other advantages of the present invention will be readily appreciated and apparent to those skilled in the art as the same becomes better understood by reference to the following detailed description when considered in connection with the accompanying drawings wherein:

Figure 1 is a perspective view of the intradermal delivery device of the present invention;

Figure 2 is a side sectional view of the intradermal delivery device showing the limiter in the first position;

Figure 3 is a side sectional view of the intradermal delivery device showing the limiter in the second position;

Figure 4 is a side sectional view of the intradermal delivery device showing the limiter in the third position; and

Figure 5 is a side sectional view of the intradermal delivery device showing the limiter in the fourth position.

### DETAILED DESCRIPTION OF THE PREFERRED EMBODIMENT

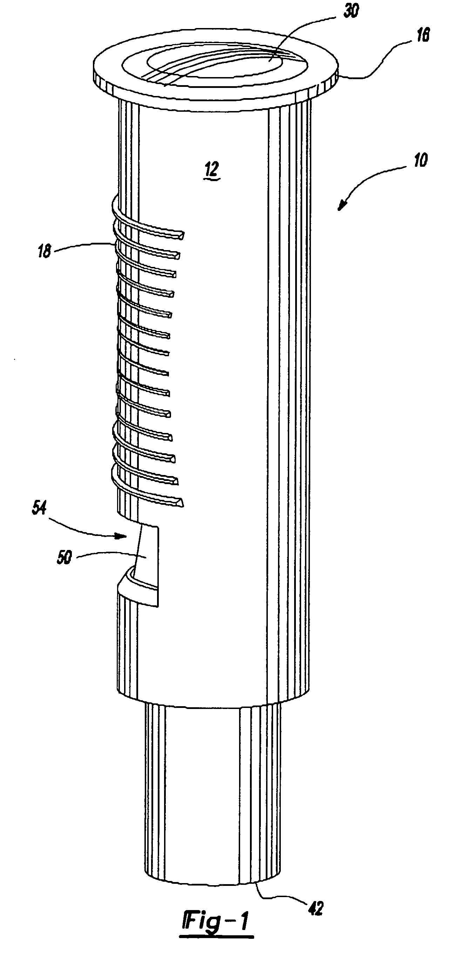

An intradermal delivery device is generally shown in Figures 1 to 5 at 10. The device includes a sleeve 12 having a first proximate end 14 and a second distal end 16. A plurality of ribs 18 are disposed on the outside surface of the sleeve 12 to assist gripping the sleeve 12, the purpose of which will be further evident below. A limiter 20 is slidably inserted through the first end 14 of the sleeve 12.

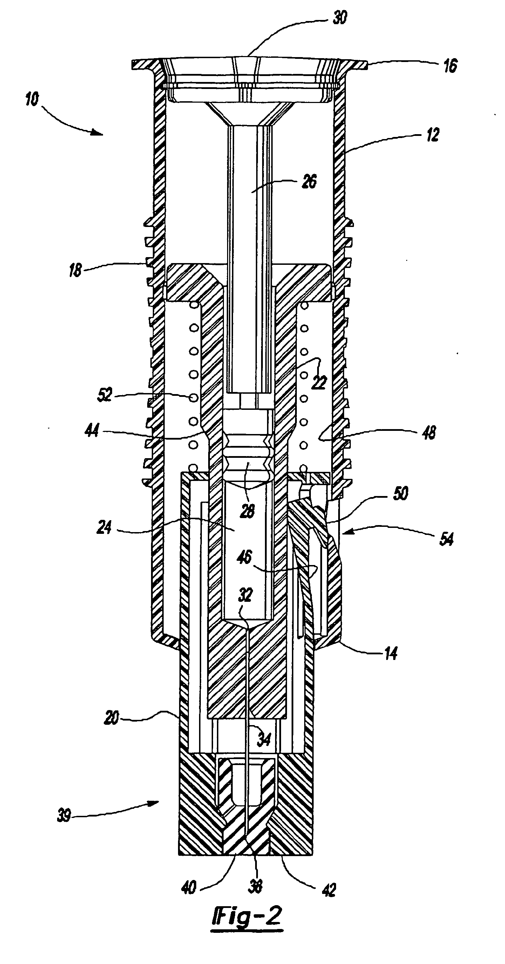

Referring to Figure 2, a prefillable container 22 is disposed inside the sleeve 12. The prefillable container 22 includes a reservoir 24 adapted to store a substance intended for intradermal delivery into the skin of an animal. The substance comprises drugs, vaccines, and the like that are known to be absorbed significantly better in the dermis layer of the skin of the animal as opposed to the subcutaneous region of the animal.

Also, the substance intradermally delivered in accordance with the method of the present invention is selected from the group consisting of drugs, vaccines and the like used in the prevention, diagnosis, alleviation, treatment, or cure of disease, with the drugs including Alpha-1 anti-trypsin, Anti-Angiogenesis agents, Antisense, butorphanol, Calcitonin and analogs, Ceredase, COX-II inhibitors, dermatological agents, dihydroergotamine, Dopamine agonists and antagonists, Enkephalins and other opioid peptides, Epidermal growth factors, Erythropoietin and analogs, Follicle stimulating hormone, G-CSF, Glucagon, GM-CSF, granisetron, Growth hormone and analogs (including growth hormone releasing hormone), Growth hormone antagonists, Hirudin and Hirudin analogs such as hirulog, IgE suppressors, Insulin, insulinotropin and analogs, Insulin-like growth factors, Interferons, Interleukins, Leutenizing hormone, Leutenizing hormone releasing hormone and analogs, Low molecular weight heparin, M-CSF, metoclopramide, Midazolam, Monoclonal antibodies, Narcotic analgesics, nicotine, Non-steroid anti-inflammatory agents, Oligosaccharides, ondansetron, Parathyroid hormone and analogs, Parathyroid hormone antagonists, Prostaglandin antagonists, Prostaglandins, Recombinant soluble receptors, scopolamine, Serotonin agonists and antagonists, Sildenafil, Terbutaline, Thrombolytics, Tissue plasminogen activators, TNF - , and TNF - antagonist, the vaccines, with or without carriers/adjuvants, including prophylactics and therapeutic antigens (including but not limited to subunit protein, peptide and polysaccharide, polysaccharide conjugates, toxoids, genetic based vaccines, live attenuated, reassortant, inactivated, whole cells, viral and bacterial vectors) in connection with, addiction, arthritis, cholera, cocaine addiction, diphtheria, tetanus, HIB, Lyme disease, meningococcus, measles, mumps, rubella, varicella, yellow fever, Respiratory syncytial virus, tick borne japanese encephalitis, pneumococcus, streptococcus, typhoid, influenza, hepatitis, including hepatitis A, B, C and E, otitis media, rabies, polio, HIV, parainfluenza, rotavirus, Epstein Barr Virus, CMV, chlamydia, non-typeable haemophilus, moraxella catarrhalis, human papilloma virus, tuberculosis including BCG, gonorrhoea, asthma, atheroschlerosis malaria, E-coli, Alzheimers, H. Pylori, salmonella, diabetes, cancer, herpes simplex, human papilloma and the like other substances including all of the major therapeutics such as agents for the common cold, Anti-addiction, anti-allergy, anti-emetics, anti-obesity, antiosteoporeteic, anti-infectives, analgesics, anesthetics, anorexics, antiarthritics, antiasthmatic agents, anticonvulsants, anti-depressants, antidiabetic agents, antihistamines, anti-inflammatory agents, antimigraine preparations, antimotion sickness preparations, antinauseants, antineoplastics, antiparkinsonism drugs, antipruritics, antipsychotics, antipyretics, anticholinergics, benzodiazepine antagonists, vasodilators, including general, coronary, peripheral and cerebral, bone stimulating agents, central nervous system stimulants, hormones, hypnotics, immunosuppressives, muscle relaxants, parasympatholytics, parasympathomimetrics, prostaglandins, proteins, peptides, polypeptides and other macromolecules, psychostimulants, sedatives, sexual hypofunction and tranquilizers and major diagnostics such as tuberculin and other hypersensitivity agents.

The present invention also includes the use of the above substances in the preparation of a filled device for making an intradermal injection into the skin of an animal. Accordingly, as disclosed in the co-pending U.S. patent application, Serial No. 09/835,243, filed April 13, 2001, entitled "METHOD OF INTRADERMAL INJECTING SUBSTANCES".

A plunger 26 is slidably received in the reservoir 24 through the open end. A stopper 28 seals the reservoir 24 and is selectively movable by the plunger 26 for expelling the substance from the prefillable container reservoir 22. A manual activation flange or button 30 is disposed at an opposite end of the plunger 26 from the stopper 28. The flange 30 is fixedly attached to the second end 16 of the sleeve 12, the purpose of which will be explained below.

The prefillable container includes an outlet port 32 through which the substance is expelled from the reservoir 24. A needle cannula 34 is affixed to the prefillable container 22 and is in fluid communication with the outlet port 32. The needle cannula 34 may be attached to the prefillable container 22 with an adhesive, a mechanical, interference fit, or an equivalent known method of attachment.

The needle cannula 34 includes a forward tip 36 that is adapted to administer an intradermal injection. Preferably, the forward tip 36 includes a beveled edge 38 ranging in length from approximately 0.8 mm to 1.0 mm. More preferably, the beveled edge 38 includes a length of approximately 0.9 mm. A standard beveled tip length ranges from approximately 1.3 mm to 1.6 mm. The reduced length of the present beveled edge 38 reduces the potential of the needle cannula 34 piercing through the dermis layer of the skin of the animal and resulting in the substance from the reservoir 24 being injected into the subcutaneous region of the animal.

The prefillable container reservoir 22 is slidably received within the limiter 20. The limiter includes a pierceable elastomeric insert 40 into which the forward tip 36 of the needle cannula 34 is embedded (Figure 1) when the limiter is in a first position 39. Preferably, the insert 40 is formed from an elastomer or a thermal plastic elastomer. The insert 40 seals the needle cannula 34 preventing the prefilled substance from leaking from the reservoir 24 through the cannula 34 prior to administering the intradermal injection.

The elastomeric insert 40 is centrally located in a skin engaging surface 42 on the limiter 20. The skin engaging surface 42 is generally planar, annular and continuous and provides a stable placement of the delivery device 10 against an animal skin. The width of the annular skin engaging surface is preferably at least 5 mm. Further, the planar skin engaging surface 42 extends generally perpendicular to the axis of the needle cannula.

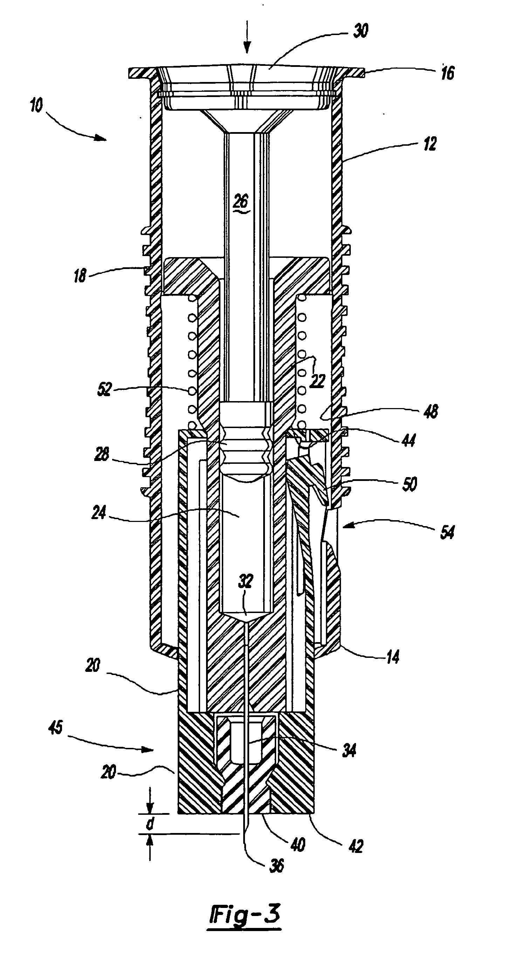

As shown in Figure 3, the forward tip 36 of the needle cannula 34 extends beyond the skin engaging surface 42 a length "d" determined to facilitate administering an intradermal injection. The forward tip 36 extends past the skin engaging surface 42 a distance of approximately 0.5 mm. to 3 mm preferably 1.0 mm to 2.0 mm, and most preferably 1.5 mm ± 0.2 to 0.3 mm. The preferred needle size is a small gauge hypodermic needle, commonly known as a 30 Gauge or a 31 Gauge needle.

As shown in Figures 2 through 5, the prefillable container 22 is slidably received within the limiter 20 inside the sleeve 12. To force the forward tip 36 of the needle cannula 34 through the insert 40, force is applied to the activation flange 30 of the plunger 26. Accordingly, the limiter 20 slides into the sleeve 12 reducing the length the limiter 20 is exposed outside the sleeve 12. The prefillable container 22 is stationary relative to the sleeve 12 when the limiter 20 is moving. Thus, when the limiter 20 moves relative to the sleeve 12, the limiter 20 is also moving relative to the prefillable container 22. The prefillable container 22 includes a stop 44 represented in the Figures as the surface of the prefillable container 22 having an increased diameter. The stop 44 terminates movement of the limiter 20 in a second position 45 as shown in Figure 3 exposing a predetermined length "d" of the needle cannula 34 relative to the skin engaging surface 42.

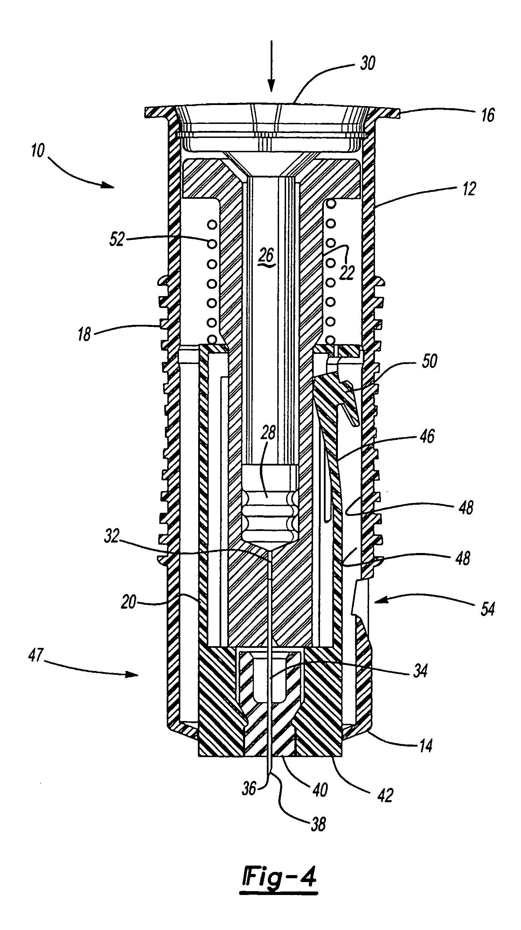

Referring to Figure 4, continued force on the actuation flange 30 drives the plunger 26 inwardly of the prefillable container 22 expelling the substance from the reservoir 24 through the needle cannula 34 into the skin of the animal, thereby moving the intradermal delivery device 10 from the second position 45 to a third position 47

As shown in Figures 2 through 5, the limiter 20 includes a locking arm 46 oriented generally parallel to the prefillable container 22 and being biased outwardly from the limiter 20 toward an inner surface 48 of the sleeve 12. The locking arm 46 includes a catch represented in the Figures as a hook 50 that slides freely inside the sleeve 12 when moving the limiter 20 from the first position 39 through the third position 47.

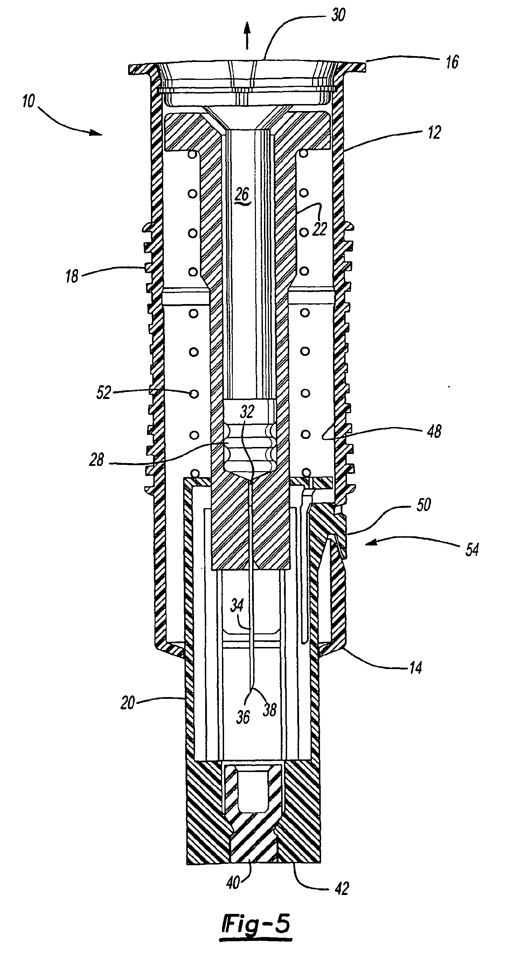

Referring to Figure 5, subsequent to administering a intradermal injection, the limiter 20 is movable from the third position 47 to a fourth position 49, to enclose the needle cannula 34 inside the limiter 20. A spring 52 is optionally disposed inside the sleeve 12 and biases the limiter 20 outwardly of the sleeve 12. Preferably, the spring 52 is coiled around the prefillable container 22 and is compressed between the activation flange 30 and the limiter 20. Upon releasing the force from the activation flange 30, the spring 52 forces the limiter 20 outwardly of the sleeve 12, which withdraws the needle cannula 34 back into the limiter 20, thereby moving the device from the third position 47 to the fourth position 49. When the limiter 20 has reached the fourth position, the hook 50 engages a slot 54 disposed in the sleeve 12 in a manner that locks the limiter 20 to prevent the limiter from being moved from the fourth position 49 back to the third position 47 in which the needle tip would be exposed.

The invention has been described in an illustrative manner, and it is to be understood that the terminology which has been used is intended to be in the nature of words of description rather than of limitation.

Obviously, many modifications and variations of the present invention are possible in light of the above teachings. It is, therefore, to be understood that within the scope of the appended claims, wherein reference numerals are merely for convenience and are not to be in any way limiting, the invention may be practiced otherwise than as specifically described.

## Claims

1. A prefillable intradermal injector for use in intradermally injecting substances into the skin nof an animal comprising:
a prefillable reservoir (24) adapted to store a substance and having an outlet port (32) at a first end enabling the substance to be expelled from said reservoir and a stopper (28) in a second open end;
a needle cannula (34) defining an axis and said needle cannula in fluid communication with said reservoir (24) to receive the substance from said outlet port (32) and having a forward tip (36) adapted to penetrate the skin of an animal;
a limiter (20) surrounding said needle cannula, said limiter including a generally flat skin engaging surface (42) extending in a plane generally perpendicular to an axis of said needle cannula, said limiter limiting penetration of said forward tip of said needle cannula into the dermis layer of the skin of the animal; and
a sleeve (12) having a first end (14) and a second end (16),
wherein said sleeve (12) surrounds and is slidable relative to said reservoir (24) along said axis and said sleeve is fixed relative to said stopper (28) whereby movement of said sleeve causes said stopper to move through said reservoir to expel said substance from said reservoir through said needle cannula.

2. The prefillable intradermal injector as defined in claim 1, wherein said limiter (20) is moveable axially relative to said needle cannula (34) from a retracted position wherein said forward tip (36) of said needle cannula extends through said limiter to an extended position wherein said needle cannula forward tip is enclosed within said limiter.

3. The prefillable intradermal injector as defined in claim 2, wherein said forward tip (36) of said needle cannula (34) extends through said limiter (20) beyond said skin engaging surface no more than about 0,5 mm to about 3,0 mm in said retracted position of said limiter.

4. The prefillable intradermal injector as defined in claim 1, wherein said limiter (20) is moveable axially relative to said reservoir (24) from a first position wherein said forward tip (36) of said needle is located within said limiter to a second position wherein said needle extends through said limiter beyond said skin engaging surface (42) no more than from about 0,5 to 3,0 mm.

5. A prefillable intradermal injector as set forth in claim 4, wherein said limiter (20) includes an axial opening therethrough receiving said forward tip (36) of said needle cannula (34) and said opening includes pierceable insert (40) sealing said axial opening.

6. The prefillable intradermal injector as set forth in claim 4, wherein said forward tip of said needle cannula extends beyond said skin engaging surface (42) no more than from about 1,0 to 2,0 mm.

7. The prefillable intradermal injector as set forth in claim 4, wherein said forward tip (36) of said needle cannula (34) extending beyond said skin engaging surface (42) about 1,5 mm ± 0,2 to 0,3 mm.

8. The prefillable intradermal injector as set forth in claim 5, wherein said forward tip (36) of said needle cannula (34) pierces said insert (40) when said limiter (20) is located in said second position thereby exposing said forward tip enabling administration of the intradermal injection.

9. A prefillable intradermal injector as set forth in claim 2, wherein said limiter includes a locking arm (46) and said sleeve (12) includes a catch (54) engaging said locking arm when said limiter is located in said extended position enclosing said forward tip of said needle cannula.

10. A prefillable intradermal injector as set forth in claim 9, wherein said locking arm (46) includes a hook (50) and said catch (54) includes a slot disposed in said sleeve (12), said hook engaging said slot when said limiter is located in said extended position.

11. A prefillable intradermal injector as set forth in claim 4, wherein said prefillable reservoir (24) includes a stop (44) to terminate movement of said limiter (20) at said second position when moving said limiter between said first position (39) and said second position (45).

12. A prefillable intradermal injector as set forth in claim 2, further comprising a spring (52) biasing said limiter (20) to move from said retracted position to said extended position.

13. A prefillable intradermal injector as set forth in claim 12, wherein said spring (52) is coiled around said reservoir (24).

14. A prefillable intradermal injector as set forth in claim 1, wherein said sleeve (12) includes a stopper engaging portion.

15. A prefillable intradermal injector as set forth in claim 14, wherein said stopper engaging portion includes a plunger rod (26) operably connected to said second end of said sleeve (12).

16. A prefillable intradermal injector, comprising:
a reservoir (24) prefillable with a substance having a generally closed proximal end and an open distal end;
a needle cannula (37) having a distal end in fluid communication with said substance in said reservoir (24) extending through said proximal end and an exposed forward tip (36);
a stopper (28) slideably received in said open distal end of said reservoir (24) moveable in said reservoir to expel said substance through said forward tip (36) of said needle cannula;
a sleeve (12) surrounding said reservoir (24) operably fixed to said stopper (28) whereby movement of said sleeve relative to said reservoir moves said stopper through said reservoir;
a limiter (20) having a distal end slideably supported around said reservoir (24) and moveable relative to said reservoir and a generally closed proximal end including a flat skin engaging surface (42) having an opening therethrough receiving said forward tip (36) of said needle cannula therethrough as said limiter moves relative to said reservoir and said limiter (20) limiting extension of said forward tip of said needle cannula beyond said skin engaging surface a distance of more than 3.0 mm.

17. The prefillable intradermal injector as set forth in claim 16, wherein said proximal end of said reservoir (24) engages a surface of said limiter (20) as said limiter is moved toward said reservoir, thereby limiting movement of said limiter relative to said reservoir.

18. The prefillable intradermal injector as set forth in claim 16, wherein said opening through said proximal end of said limiter (20) includes a pierceable closure (40) and said forward tip (36) of said needle cannula (34) pierces said closure as said limiter is moved toward said reservoir.

19. The prefillable intradermal injector as set forth in claim 16, wherein said forward tip (36) of needle cannula (34) extends through said limiter beyond said skin engaging surface no more than from about 1.0 to 2.0 mm.

20. The prefillable intradermal injector as set forth in claim 19, wherein said forward tip of said needle cannula extends through said limiter 1.5 ± 0.2 to 0.3 mm.

21. The prefillable intradermal injector as set forth in claim 16, further comprising a spring (52) biased between said reservoir (24) and said limiter (20) moving said limiter to an extended position enclosing said forward tip (36) of said needle cannula (34) following injection.

## Patentansprüche

1. Vorfüllbare intradermale Injektionsvorrichtung zum intradermalen Injizieren von Substanzen in die Haut eines Lebewesens, mit:
einem vorfüllbaren Reservoir (24), das zur Aufnahme einer Substanz geeignet ist und an einem ersten Ende einen Ausgabekanal (32), über den die Substanz aus dem Reservoir herausgedrückt werden kann, und an einem offenen zweiten Ende einen Stopfen (28) aufweist;
einer Nadelkanüle (34), die eine Achse definiert, wobei sich die Nadelkanüle in Flüssigkeitsverbindung mit dem Reservoir (24) befindet, um die Substanz aus dem Ausgabekanal (32) zu erhalten, und ein Vorderende (36) aufweist, das zum Eindringen in die Haut eines Lebewesens in der Lage ist;
einem die Nadelkanüle umgebenden Begrenzerteil (20) mit einer im Wesentlichen flachen Hautanlagefläche (42), die in einer sich im Wesentlichen rechtwinklig zu der Achse der Nadelkanüle erstreckenden Ebene verläuft, wobei das Begrenzerteil das Eindringen der Vorderendes der Nadelkanüle in die Lederhautschicht der Haut des Lebewesens begrenzt; und
einer Hülse (12) mit einem ersten Ende (14) und einem zweiten Ende (16),
wobei die Hülse (12) das Reservoir (24) umgibt und relativ zu diesem entlang der besagten Achse gleitbar ist und die Hülse relativ zu dem Stopfen (28) fixiert ist, wodurch eine Bewegung der Hülse den Stopfen zur Bewegung durch das Reservoir veranlasst, um die Substanz aus dem Reservoir durch die Nadelkanüle herauszudrücken.

2. Vorfüllbare intradermale Injektionsvorrichtung nach Anspruch 1, bei der das Begrenzerteil (20) relativ zu der Nadelkanüle (34) axial aus einer eingefahrenen Position, in der sich das Vorderende (36) der Nadelkanüle durch das Begrenzerteil hindurch erstreckt, in eine ausgefahrene Position bewegbar ist, in der das Nadelkanülen-Vorderende in dem Begrenzerteil eingeschlossen ist.

3. Vorfüllbare intradermale Injektionsvorrichtung nach Anspruch 2, bei der sich in der eingefahrenen Position des Begrenzerteils das Vorderende (36) der Nadelkanüle (34) durch das Begrenzerteil (20) hindurch um höchstens ungefähr 0,5 mm bis ungefähr 3,0 mm über die Hautanlagerfläche hinaus erstreckt.

4. Vorfüllbare intradermale Injektionsvorrichtung nach Anspruch 1, bei der das Begrenzerteil (20) relativ zu dem Reservoir (24) axial aus einer ersten Position, in der das Vorderende (36) der Nadel in dem Begrenzerteil angeordnet ist, in eine zweite Position bewegbar ist, in der sich die Nadel durch das Begrenzerteil hindurch um höchstens ungefähr 0,5 mm bis ungefähr 3,0 mm über die Hautanlagefläche (42) hinaus erstreckt.

5. Vorfüllbare intradermale Injektionsvorrichtung nach Anspruch 4, bei der das Begrenzerteil (20) eine axiale Durchgangsöffnung aufweist, welche das Vorderende (36) der Nadelkanüle (34) aufnimmt, wobei die Öffnung einen durchstechbaren Einsatz (40) aufweist, der die axiale Öffnung abdichtet.

6. Vorfüllbare intradermale Injektionsvorrichtung nach Anspruch 4, bei der sich das Vorderende der Nadelkanüle um höchstens ungefähr 1,0 mm bis 2,0 mm über die Hautanlagefläche (42) hinaus erstreckt.

7. Vorfüllbare intradermale Injektionsvorrichtung nach Anspruch 4, bei der sich das Vorderende (36) der Nadelkanüle (34) um höchstens ungefähr 1,5 mm ± 0,2 bis 0,3 mm über die Hautanlagefläche (42) hinaus erstreckt.

8. Vorfüllbare intradermale Injektionsvorrichtung nach Anspruch 5, bei der, wenn das Begrenzerteil (20) in der zweiten Position angeordnet ist, das Vorderende (36) der Nadelkanüle (34) den Einsatz (40) durchsticht und dadurch das Vorderende freilegt und die Verabreichung der intradermalen Injektion ermöglicht.

9. Vorfüllbare intradermale Injektionsvorrichtung nach Anspruch 2, bei der das Begrenzerteil einen Verriegelungsarm (46) aufweist und die Hülse (12) eine Sperrvorrichtung (54) aufweist, die an dem Verriegelungsarm angreift, wenn sich das Begrenzerteil in der ausgefahrenen Position befindet, in der es das Vorderende der Nadelkanüle einschließt.

10. Vorfüllbare intradermale Injektionsvorrichtung nach Anspruch 9, bei der der Verriegelungsarm (46) einen Haken (50) aufweist und die Sperrvorrichtung (54) einen in der Hülse (12) ausgebildeten Schlitz aufweist, wobei der Haken in den Schlitz eingreift, wenn sich das Begrenzerteil in der ausgefahrenen Position befindet.

11. Vorfüllbare intradermale Injektionsvorrichtung nach Anspruch 4, bei der das vorfüllbare Reservoir (24) ein Anschlagteil (44) aufweist, um die Bewegung des Begrenzerteils (20) an der zweiten Position zu beenden, wenn das Begrenzerteil zwischen der ersten Position (39) und der zweiten Position (45) bewegt wird.

12. Vorfüllbare intradermale Injektionsvorrichtung nach Anspruch 2, ferner mit einer Feder (52), die das Begrenzerteil (20) zur Bewegung aus der eingefahrenen Position in die ausgefahrene Position vorspannt.

13. Vorfüllbare intradermale Injektionsvorrichtung nach Anspruch 12, bei der die Feder (52) um das Reservoir (24) gewunden ist.

14. Vorfüllbare intradermale Injektionsvorrichtung nach Anspruch 1, bei der die Hülse (12) einen Stopfenangriffsteil aufweist.

15. Vorfüllbare intradermale Injektionsvorrichtung nach Anspruch 14 bei der der Stopfenangriffsteil eine Kolbenstange (26) aufweist, die mit dem zweiten Ende der Hülse (12) betriebsmäßig verbunden ist.

16. Vorfüllbare intradermale Injektionsvorrichtung mit:
einem mit einer Substanz vorfüllbaren Reservoir (24) mit einem im Wesentlichen geschlossenen proximalen Ende und einem offenen distalen Ende;
einer Nadelkanüle (37) mit einem distalen Ende, das sich in Flüssigkeitsverbindung mit der in dem Reservoir (24) enthaltenen Substanz befindet und das sich durch das besagte proximale Ende erstreckt, und mit einem freiliegenden Vorderende (36);
einem gleitbar in dem offenen distalen Ende des Reservoirs (24) aufgenommenen Stopfen (28), der in dem Reservoir bewegbar ist, um die Substanz durch das Vorderende (36) der Nadelkanüle hindurch herauszudrücken;
einer das Reservoir (24) umgebenden Hülse (12), die betriebsmäßig mit dem Stopfen (28) verbunden ist, wodurch eine Bewegung der Hülse relativ zu dem Reservoir eine Bewegung des Stopfens durch das Reservoir bewirkt;
einem Begrenzerteil (20) mit einem distalen Ende, das gleitbar um das Reservoir (24) herum gehalten und relativ zu dem Reservoir bewegbar ist, und einem im Wesentlichen geschlossenen proximalen Ende mit einer flachen Hautanlagefläche (42), die eine Durchgangsöffnung aufweist, welche das Vorderende (36) der Nadelkanüle durch sie hindurch aufnimmt, wenn sich das Begrenzerteil relativ zu dem Reservoir bewegt, wobei das Begrenzerteil (20) die Ausfahrbewegung des Vorderendes der Nadelkanüle über die Hautanlagefläche hinaus auf eine Distanz von höchstens 3,0 mm begrenzt.

17. Vorfüllbare intradermale Injektionsvorrichtung nach Anspruch 16, bei der das proximale Ende des Reservoirs (24) an einer Fläche des Begrenzerteils (20) angreift, wenn das Begrenzerteil zu dem Reservoir hin bewegt wird, wodurch die Bewegung des Begrenzerteils relativ zu dem Reservoir begrenzt wird.

18. Vorfüllbare intradermale Injektionsvorrichtung nach Anspruch 16, bei der die durch das proximale Ende des Begrenzerteils (20) verlaufende Öffnung einen durchstechbaren Verschluss (40) aufweist und das Vorderende (36) der Nadelkanüle (34) den Verschluss durchsticht, wenn sich das Begrenzerteil zu dem Reservoir bewegt.

19. Vorfüllbare intradermale Injektionsvorrichtung nach Anspruch 16, bei der sich das Vorderende (36) der Nadelkanüle (34) durch das Begrenzerteil hindurch um höchstens ungefähr 1,0 mm bis 2,0 mm über die Hautanlagefläche hinaus erstreckt.

20. Vorfüllbare intradermale Injektionsvorrichtung nach Anspruch 19, bei der sich das Vorderende der Nadelkanüle um höchstens ungefähr 1,5 mm ± 0,2 bis 0,3 mm durch das Begrenzerteil erstreckt.

21. Vorfüllbare intradermale Injektionsvorrichtung nach Anspruch 16, ferner mit einer unter Vorspannung zwischen dem Reservoir (24) und dem Begrenzerteil (20) angeordneten Feder (52), die nach der Injektion das Begrenzerteil in eine ausgefahrene Position bewegt, in der dieses das Vorderende (36) der Nadelkanüle (34) umschließt.

## Revendications

1. Injecteur intradermique à préremplissage, destiné à être utilisé pour injecter par voie intradermique des substances à l'intérieur de la peau d'un animal, comprenant :
un réservoir à préremplissage (24) adapté pour stocker une substance et présentant un orifice de sortie (32) à une première extrémité permettant d'expulser la substance dudit réservoir et un bouchon (28) dans une seconde extrémité ouverte ;
une canule d'aiguille (34) définissant un axe et ladite canule d'aiguille étant en communication fluidique avec ledit réservoir (24) pour recevoir la substance dudit orifice de sortie (32) et présentant une pointe avant (36) adaptée pour pénétrer dans la peau d'un animal ;
un limiteur (20) entourant ladite canule d'aiguille, ledit limiteur comportant une surface de mise en prise avec la peau (42) généralement plate s'étendant dans un plan généralement perpendiculaire à un axe de ladite canule d'aiguille, ledit limiteur limitant la pénétration de ladite pointe avant de ladite canule d'aiguille à l'intérieur de la couche dermique de la peau de l'animal ; et
une gaine (12) présentant une première extrémité (14) et une seconde extrémité (16),
dans lequel ladite gaine (12) entoure ledit réservoir (24) et peut glisser par rapport à celui-ci le long dudit axe et ladite gaine est fixe par rapport audit bouchon (28), le déplacement de ladite gaine faisant déplacer ledit bouchon à travers ledit réservoir pour expulser ladite substance dudit réservoir à travers ladite canule d'aiguille.

2. Injecteur intradermique à préremplissage selon la revendication 1, dans lequel ledit limiteur (20) est mobile axialement par rapport à ladite canule d'aiguille (34) depuis une position rétractée dans laquelle ladite pointe avant (36) de ladite canule d'aiguille s'étend à travers ledit limiteur vers une position étendue dans laquelle ladite pointe avant de canule d'aiguille est enfermée à l'intérieur dudit limiteur.

3. Injecteur intradermique à préremplissage selon la revendication 2, dans lequel ladite pointe avant (36) de ladite canule d'aiguille (34) s'étend à travers ledit limiteur (20) au-delà de ladite surface de mise en prise avec la peau sur pas plus d'environ 0,5 mm à environ 3,0 mm dans ladite position rétractée dudit limiteur.

4. Injecteur intradermique à préremplissage selon la revendication 1, dans lequel ledit limiteur (20) est mobile axialement par rapport audit réservoir (24) depuis une première position dans laquelle ladite pointe avant (36) de ladite aiguille est positionnée à l'intérieur dudit limiteur vers une seconde position dans laquelle ladite aiguille s'étend à travers ledit limiteur au-delà de ladite surface de mise en prise avec la peau (42) sur pas plus d'environ 0,5 à 3,0 mm.

5. Injecteur intradermique à préremplissage selon la revendication 4, dans lequel ledit limiteur (20) comporte une ouverture axiale à travers celui-ci recevant ladite pointe avant (36) de ladite canule d'aiguille (34) et ladite ouverture comporte un insert perçable (40) étanchéifiant ladite ouverture axiale.

6. Injecteur intradermique à préremplissage selon la revendication 4, dans lequel ladite pointe avant de ladite canule d'aiguille s'étend au-delà de ladite surface de mise en prise avec la peau (42) sur pas plus d'environ 1,0 à 2,0 mm.

7. Injecteur intradermique à préremplissage selon la revendication 4, dans lequel ladite pointe avant (36) de ladite canule d'aiguille (34) s'étend au-delà de ladite surface de mise en prise avec la peau (42) sur environ 1,5 mm±0,2 à 0,3 mm.

8. Injecteur intradermique à préremplissage selon la revendication 5, dans lequel ladite pointe avant (36) de ladite canule d'aiguille (34) perce ledit insert (40) lorsque ledit limiteur (20) est positionné dans ladite seconde position exposant de ce fait ladite pointe avant, permettant l'administration de l'injection intradermique.

9. Injecteur intradermique à préremplissage selon la revendication 2, dans lequel ledit limiteur comporte un bras de verrouillage (46) et ladite gaine (12) comporte un cliquet (54) se mettant en prise avec ledit bras de verrouillage lorsque ledit limiteur est positionné dans ladite position étendue enfermant ladite pointe avant de ladite canule d'aiguille.

10. Injecteur intradermique à préremplissage selon la revendication 9, dans lequel ledit bras de verrouillage (46) comporte un crochet (50) et ledit cliquet (54) comporte une fente disposée dans ladite gaine (12), ledit crochet se mettant en prise avec ladite fente lorsque ledit limiteur est positionné dans ladite position étendue.

11. Injecteur intradermique à préremplissage selon la revendication 4, dans lequel ledit réservoir à préremplissage (24) comporte une butée (44) pour terminer le déplacement dudit limiteur (20) dans ladite seconde position lorsque ledit limiteur est déplacé entre ladite première position (39) et ladite seconde position (45).

12. Injecteur intradermique à préremplissage selon la revendication 2, comprenant en outre un ressort (52) sollicitant ledit limiteur (20) pour qu'il se déplace de ladite position rétractée à ladite position étendue.

13. Injecteur intradermique à préremplissage selon la revendication 12, dans lequel ledit ressort (52) est enroulé autour dudit réservoir (24).

14. Injecteur intradermique à préremplissage selon la revendication 1, dans lequel ladite gaine (12) comporte une partie de mise en prise avec le bouchon.

15. Injecteur intradermique à préremplissage selon la revendication 14, dans lequel ladite partie de mise en prise avec le bouchon comporte une tige de piston (26) connectée de manière opérationnelle à ladite seconde extrémité de ladite gaine (12).

16. Injecteur intradermique à préremplissage, comprenant :
un réservoir (24) pouvant être prérempli avec une substance présentant une extrémité proximale généralement fermée et une extrémité distale ouverte ;
une canule d'aiguille (34) présentant une extrémité distale en communication fluidique avec ladite substance dans ledit réservoir (24) s'étendant à travers ladite extrémité proximale et une pointe avant exposée (36) ;
un bouchon (28) reçu de manière coulissante dans ladite extrémité distale ouverte dudit réservoir (24) mobile dans ledit réservoir pour expulser ladite substance à travers ladite pointe avant (36) de ladite canule d'aiguille ;
une gaine (12) entourant ledit réservoir (24) fixée de manière opérationnelle audit bouchon (28), le déplacement de ladite gaine par rapport audit réservoir déplaçant ledit bouchon à travers ledit réservoir ;
un limiteur (20) présentant une extrémité distale supportée de manière coulissante autour dudit réservoir (24) et mobile par rapport audit réservoir et une extrémité proximale généralement fermée comportant une surface de mise en prise avec la peau plate (42) présentant une ouverture à travers celle-ci recevant ladite pointe avant (36) de ladite canule d'aiguille à travers celle-ci à mesure que ledit limiteur se déplace par rapport audit réservoir et ledit limiteur (20) limitant l'extension de ladite pointe avant de ladite canule d'aiguille au-delà de ladite surface de mise en prise avec la peau suivant une distance supérieure à 3,0 mm.

17. Injecteur intradermique à préremplissage selon la revendication 16, dans lequel ladite extrémité proximale dudit réservoir (24) se met en prise avec une surface dudit limiteur (20) à mesure que ledit limiteur est déplacé en direction dudit réservoir, limitant de ce fait le déplacement dudit limiteur par rapport audit réservoir.

18. Injecteur intradermique à préremplissage selon la revendication 16, dans lequel ladite ouverture à travers ladite extrémité proximale dudit limiteur (20) comporte une fermeture perçable (40) et ladite pointe avant (36) de ladite canule d'aiguille (34) perce ladite fermeture à mesure que ledit limiteur est déplacé en direction dudit réservoir.

19. Injecteur intradermique à préremplissage selon la revendication 16, dans lequel ladite pointe avant (36) de la canule d'aiguille (34) s'étend à travers ledit limiteur au-delà de ladite surface de mise en prise avec la peau sur pas plus d'environ 1,0 à 2,0 mm.

20. Injecteur intradermique à préremplissage selon la revendication 19, dans lequel ladite pointe avant de ladite canule d'aiguille s'étend à travers ledit limiteur sur 1,5±0,2 à 0,3 mm.

21. Injecteur intradermique à préremplissage selon la revendication 16, comprenant en outre un ressort (52) sollicité entre ledit réservoir (24) et ledit limiteur (20) déplaçant ledit limiteur vers une position étendue enfermant ladite pointe avant (36) de ladite canule d'aiguille (34) à la suite de l'injection.
